(19) 

Europäisches
Patentamt
European
Patent Office
Office européen
des brevets

(11) **EP 4 502 016 A1**

(12) **EUROPEAN PATENT APPLICATION**
published in accordance with Art. 153(4) EPC

(43) Date of publication:
**05.02.2025 Bulletin 2025/06**

(21) Application number: **23780501.5**

(22) Date of filing: **28.03.2023**

(51) International Patent Classification (IPC):
*C08J 3/12* (2006.01)      *C08J 3/24* (2006.01)
*C08K 5/00* (2006.01)      *C08L 101/14* (2006.01)
*A61F 13/53* (2006.01)     *C08K 3/30* (2006.01)
*A61L 15/24* (2006.01)     *A61L 15/60* (2006.01)

(52) Cooperative Patent Classification (CPC):
**A61F 13/53; A61L 15/24; A61L 15/60; C08J 3/12;
C08J 3/24; C08K 3/30; C08K 5/00; C08L 101/14**

(86) International application number:
**PCT/JP2023/012460**

(87) International publication number:
**WO 2023/190492 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.03.2022 JP 2022057398**

(71) Applicant: **Sumitomo Seika Chemicals Co., Ltd.
Kako-gun, Hyogo 675-0145 (JP)**

(72) Inventor: **GOGA, Yuki
Himeji-shi, Hyogo 672-8076 (JP)**

(74) Representative: **Mewburn Ellis LLP
Aurora Building
Counterslip
Bristol BS1 6BX (GB)**

(54) **METHOD FOR PRODUCING WATER-ABSORBING RESIN COMPOSITION**

(57)     Provided is a method for producing a water-absorbing resin composition, the method imparting high gel stability, suppressing an increase in the amount of water-absorbing resin particles that are dissolved by a sulfite-based compound, and suppressing yellowing that could occur under a high temperature and high humidity.

This method for producing a water-absorbing resin composition comprises a step for spraying an aqueous solution of a sulfite-based compound on surface-crosslinked water-absorbing resin particles, and a step for spraying an aqueous solution of an organic antioxidant on the surface-crosslinked water-absorbing resin particles.

EP 4 502 016 A1

## Description

Technical Field

The present invention relates to a method for producing a water-absorbent resin composition, and more particularly to a method for producing a water-absorbent resin composition constituting an absorbent material suitably used for hygienic materials such as disposable diapers, sanitary napkins, and incontinence pads.

Background Art

In recent years, water-absorbent resins have been widely used in the field of hygienic materials such as disposable diapers, sanitary napkins, and incontinence pads.

As such a water-absorbent resin, the crosslinked product of an acrylic acid partial neutralization salt polymer is considered to be a preferable water-absorbent resin due to its many advantages such as: the water-absorbent resin has excellent water absorption capacity; acrylic acid as a raw material thereof is easily industrially available, and the water-absorbent resin can be produced at low cost in constant quality; and the water-absorbent resin is resistant to decay and deterioration.

Citation List

Patent Literature

PTL 1: Japanese Patent Laid-open Publication No. 2005-29751

Summary of Invention

Technical Problem

The water-absorbent resin absorbs a body fluid such as human urine to generate a gel. There is a phenomenon that the gel deteriorates due to the influence of various components in the body fluid (for example, vitamin C, iron, etc. in urine). Therefore, various additives (for example, a human urine stabilizer) have been added, and the production method of the water-absorbent resin has been improved.

For example, a sulfite-based compound as a reducing agent is expected to exhibit an effect as a human urine stabilizer and enhance the gel stability of the water-absorbent resin. However, the sulfite-based compound added to the water-absorbent resin can lead to problems such as increased dissolution content of the water-absorbent resin, yellow coloration under high temperature and high humidity, and the like.

Under such circumstances, a main object of the present invention is to provide a method for producing a water-absorbent resin composition with high gel stability that suppresses an increase in the dissolution content of the water-absorbent resin and yellow coloration under high temperature and high humidity due to the sulfite-based compound.

Solution to Problem

The present inventors have conducted intensive studies in order to solve the above problems. As a result, the present inventors have found that an aqueous solution of a sulfite-based compound and an aqueous solution of an organic antioxidant are sprayed to a water-absorbent resin particle having been subjected to a surface-crosslinking process, and thus a water-absorbent resin composition with high gel stability that suppresses an increase in the dissolution content of the water-absorbent resin particle and yellow coloration under high temperature and high humidity due to the sulfite-based compound can be obtained. The present invention has been completed through further intensive studies based on such findings.

That is, the present invention provides inventions having the following configurations.

Item 1. A method for producing a water-absorbent resin composition, the method including: a step of spraying an aqueous solution of a sulfite-based compound, and a step of spraying an aqueous solution of an organic antioxidant, to a water-absorbent resin particle having been subjected to a surface-crosslinking process.

Item 2. The method for producing a water-absorbent resin composition according to Item 1, wherein the sulfite-based compound includes at least one selected from a group consisting of sodium sulfite, potassium sulfite, calcium sulfite, sodium hydrogen sulfite, potassium hydrogen sulfite, ammonium hydrogen sulfite, sodium metabisulfite, and potassium metabisulfite.

Item 3. The method for producing a water-absorbent resin composition according to Item 1 or 2, wherein the organic antioxidant includes at least one selected from a group consisting of ascorbic acids, erythorbic acids, gallic acids, protocatechuic acids, benzimidazoles, and alkylhydroxyanisoles.

Item 4. The method for producing a water-absorbent resin composition according to any one of Items 1 to 3, wherein the water-absorbent resin particle has a dissolution content of 19 parts by mass or less, the dissolution content being determined by a measurement method below.

(Dissolution content)

In a 500 mL beaker, 500 g of physiological saline is stirred with a stirring bar rotating at 600 rpm. The physiological saline has a temperature of 25°C. Into the physiological saline, 2.000 g of the water-absorbent resin particle is added, and a dispersion containing the water-absorbent resin particle is stirred for three hours. The dispersion is filtered through a 75 μm standard sieve to recover a filtrate. Eighty grams of the obtained filtrate is weighed in a weighed 100 mL beaker that has been previously subjected to constant weight-determination at 140°C. The filtrate in the beaker is heated in a hot air dryer at 140°C for 15 hours to remove moisture, and a remaining solid content is measured for mass Wa (g). A blank test is performed by above-described operation without adding the water-absorbent resin particle into the physiological saline to measure a solid content remaining in the beaker for mass Wb (g). The dissolution content is calculated by an equation below.

$$\text{Dissolution content (mass\%)} = [((Wa\text{-}Wb)/80) \times 500/2] \times 100$$

Item 5. The method for producing a water-absorbent resin composition according to any one of Items 1 to 4, wherein the sulfite-based compound is sprayed in an amount of 0.01 parts by mass or more and 3 parts by mass or less with respect to 100 parts by mass of the water-absorbent resin particle.

Item 6. The method for producing a water-absorbent resin composition according to any one of Items 1 to 5, wherein the organic antioxidant is sprayed in an amount of 0.1 parts by mass or more and 20 parts by mass or less with respect to 100 parts by mass of the sulfite-based compound.

Item 7. The method for producing a water-absorbent resin composition according to any one of Items 1 to 6, wherein the water-absorbent resin particle has a physiological saline-retention amount of 25 g/g or more and 60 g/g or less.

Item 8. The method for producing a water-absorbent resin composition according to any one of Items 1 to 7, wherein the water-absorbent resin particle has a physiological saline-absorption amount of 5 ml/g or more and 40 ml/g or less under a load of 4.14 kPa.

Advantageous Effects of Invention

According to the present invention, it is possible to provide a method for producing a water-absorbent resin composition with high gel stability that suppresses an increase in the dissolution content of the water-absorbent resin particle and yellow coloration under high temperature and high humidity due to the sulfite-based compound.

Brief Description of Drawings

[Fig. 1] Fig. 1 is a schematic view of a device for measuring a physiological saline-absorption amount under a load of 4.14 kPa.

[Fig. 2] Fig. 2 is a schematic view of a device for measuring gel strength. Description of Embodiments

In the present specification, the term "Comprising" includes "consisting essentially of" and "consisting of". In addition, in the present specification, the term "(meth) acrylic" means "acrylic or methacrylic", and the term "(meth) acrylate" means "acrylate or methacrylate". The term "water-soluble" means being soluble in water in 5 mass% or more at 25°C.

Note that, in the present specification, numerical values joined by "to" mean a numerical range including numerical values before and after the "to" as a lower limit value and an upper limit value. When a plurality of lower limit values and a plurality of upper limit values are described separately, a lower limit value and an upper limit value can be arbitrarily selected to be joined by "to".

1. Method for Producing Water-Absorbent Resin Composition

The method for producing a water-absorbent resin composition of the present invention is featured by including: a step of spraying an aqueous solution of a sulfite-based compound, and a step of spraying an aqueous solution of an organic antioxidant, to a water-absorbent resin particle having been subjected to a surface-crosslinking process. The water-absorbent resin composition produced by the method for producing a water-absorbent resin composition of the present invention with such features suppresses an increase in the dissolution content of the water-absorbent resin particle and yellow coloration under high temperature and high humidity due to the sulfite-based compound, and has high gel stability. Hereinafter, the water-absorbent resin composition of the present invention will be described in detail.

The method for producing a water-absorbent resin composition of the present invention includes: a step of spraying an aqueous solution of a sulfite-based compound (hereinafter, sometimes referred to as step (A)), and a step of spraying an aqueous solution of an organic antioxidant (hereinafter, sometimes referred to as step (B)), to a water-absorbent resin particle having been subjected to a surface-crosslinking process. The order of the step (A) and the step (B) is not particularly limited. That is, the step (A) and the step (B) may be performed in this order, the step (B) and the step (A) may be performed in this order, or the step (A) and the step (B) may be performed simultaneously. In the method for producing a water-absorbent resin composition of the present invention, for example, the step (A) of spraying an aqueous solution of a sulfite-based compound is first performed to a water-absorbent resin particle having been subjected to a surface-crosslinking process. Thus, the aqueous solution of a sulfite-based compound penetrates into the water-absorbent resin particle. Next, the step (B) of spraying an aqueous solution of an organic antioxidant is performed. Thus, the aqueous solution of an organic antioxidant further penetrates into the water-absorbent resin particle. Thereby, an increase in the dissolution content of the water-absorbent resin composition and yellow coloration under high temperature and high humidity due to the sulfite-based compound are suppressed and high gel stability due to the sulfite-based compound is exhibited. Each of the step (A) and the step (B) may be performed only once or several times.

In the steps (A) and (B), examples of the method of spraying the aqueous solution include a method using a spray nozzle device, and a method using an ultrasonic device with an ultrasonic transducer, or using a rotary atomization centrifugal spraying device. In the present invention, a method using a spray nozzle device is preferable. The spray nozzle device is not particularly limited as long as it is a device capable of spraying an aqueous solution, but is preferably a one-flow type or two-flow type spray having a spray pattern such as a flat spray, a hollow cone, or a full cone.

In the method for producing a water-absorbent resin composition of the present invention, when an attempt is made to spray an aqueous solution containing a sulfite-based compound and an organic antioxidant to a water-absorbent resin particle having been subjected to a surface-crosslinking process, the sulfite-based compound and the organic antioxidant react with each other in the aqueous solution, and the effect of the present invention cannot be exerted.

From the viewpoint of more suitably exerting the effect of the present invention, the sulfite-based compound preferably includes sodium sulfite, potassium sulfite, calcium sulfite, sodium hydrogen sulfite, potassium hydrogen sulfite, ammonium hydrogen sulfite, sodium metabisulfite, and potassium metabisulfite. The sulfite-based compound contained in the aqueous solution of a sulfite-based compound may be only one kind or two or more kinds.

From the viewpoint of more suitably exerting the effect of the present invention, in the step (A), the sulfite-based compound is sprayed in an amount of preferably 0.01 parts by mass or more, more preferably 0.1 parts by mass or more, still more preferably 0.2 parts by mass or more, and preferably 3 parts by mass or less, more preferably 2 parts by mass or less, still more preferably 1 part by mass or less, with respect to 100 parts by mass of the water-absorbent resin particle. The preferable range includes 0.01 to 3 parts by mass, 0.1 to 2 parts by mass, or the like.

In addition, from the viewpoint of more suitably exerting the effect of the present invention, the concentration of the sulfite-based compound contained in the aqueous solution of a sulfite-based compound is preferably 1 mass% or more, more preferably 5 mass% or more, still more preferably 10 mass% or more, and is preferably 25 mass% or less, more preferably 20 mass% or less. The preferable range is 1 to 25 mass%, 10 to 20 mass%, or the like.

The temperature at which the step (A) is performed is, for example, 10 to 90°C or the like. The step (A) can be performed under atmospheric pressure.

From the viewpoint of more suitably exerting the effect of the present invention, the organic antioxidant preferably includes ascorbic acids, erythorbic acids, gallic acids, protocatechuic acids, benzimidazoles, and alkylhydroxyanisoles. The organic antioxidant contained in the aqueous solution of an organic antioxidant may be only one kind or two or more kinds.

From the viewpoint of more suitably exerting the effect of the present invention, in the step (B), the organic antioxidant is sprayed in an amount of preferably 0.0001 parts by mass or more, more preferably 0.0005 parts by mass or more, still more preferably 0.001 parts by mass or more, and preferably 1 part by mass or less, more preferably 0.1 parts by mass or less, still more preferably 0.01 parts by mass or less, with respect to 100 parts by mass of the water-absorbent resin particle. The preferable range includes 0.0001 to 1 part by mass, 0.001 to 0.01 parts by mass, or the like.

From the viewpoint of more suitably exerting the effect of the present invention, in the step (B), the organic antioxidant is sprayed in an amount of preferably 0.1 parts by mass or more, more preferably 0.5 parts by mass or more, and still more preferably 1 part by mass or more, and preferably 20 parts by mass or less, more preferably 10 parts by mass or less, and still more preferably 5 parts by mass or less, with respect to 100 parts by mass of the sulfite-based compound. The preferable range includes 0.1 to 20 parts by mass, 0.5 to 10 parts by mass, and the like.

In addition, from the viewpoint of more suitably exerting the effect of the present invention, the concentration of the organic antioxidant contained in the aqueous solution of an organic antioxidant is preferably 0.001 mass% or more, and more preferably 0.01 mass% or more, and is preferably 10 mass% or less, and more preferably 5 mass% or less. The preferable range is 0.001 to 10 mass%, 0.01 to 5 mass%, or the like.

The temperature at which the step (B) is performed is, for example, 10 to 90°C or the like. The step (B) can be performed under atmospheric pressure.

The method preferably includes a step of drying the water-absorbent resin composition after the steps (A) and (B). The drying conditions are not particularly limited as long as at least a part of the moisture absorbed by the water-absorbent resin composition in the steps (A) and (B) can be removed, and for example, the water-absorbent resin composition can be dried by heating at a temperature of 100 to 150°C for about 30 to 60 minutes. The drying step is optionally provided between the step (A) and the step (B).

Next, the water-absorbent resin particle having been subjected to a surface-crosslinking process, which is subjected to the step (A) and the step (B), will be described in detail.

(Water-Absorbent Resin Particle)

The water-absorbent resin particle contained in the water-absorbent resin composition of the present invention is formed by crosslinking a polymer of a water-soluble ethylenically unsaturated monomer, that is, formed of a crosslinked polymer having a structural unit derived from a water-soluble ethylenically unsaturated monomer. The water-absorbent resin particle has been subjected to a surface-crosslinking process. As a method of the surface treatment, for example, the method described in <Surface-Crosslinking Step> described later can be adopted.

From the viewpoint of more suitably exerting the effect of the present invention, the physiological saline-retention amount of the water-absorbent resin particle is preferably 25 g/g or more, more preferably 30 g/g or more, and is preferably 60 g/g or less, more preferably 55 g/g or less, still more preferably 50 g/g or less. The preferable range is 25 to 60 g/g, 30 to 50 g/g, or the like.

In addition, from the viewpoint of more suitably exerting the effect of the present invention, the physiological saline-absorption amount of the water-absorbent resin particle under a load of 4.14 kPa is preferably 5 ml/g or more, more preferably 10 ml/g or more, still more preferably 15 ml/g or more, and is preferably 40 ml/g or less, more preferably 35 ml/g or less, still more preferably 30 ml/g or less. The preferable range is 5 to 40 ml/g, 10 to 30 ml/g, or the like.

Each of the physiological saline-retention amount and the physiological saline-absorption amount under a load of 4.14 kPa of the water-absorbent resin particle is a value measured by the method described in Examples.

From the viewpoint of more suitably exerting the effect of the present invention, the water absorption rate of the water-absorbent resin particle by the Vortex method is preferably 60 seconds or less, more preferably 50 seconds or less, and still more preferably 40 seconds or less, and is preferably 1 second or more, more preferably 3 seconds or more, and still more preferably 10 seconds or more. The preferable range is 1 to 60 seconds, 3 to 40 seconds, and the like.

The water absorption rate of the water-absorbent resin particle by the Vortex method is a value measured by the method described in Examples.

The dissolution content of the water-absorbent resin particle is the ratio of the compound eluted from the inside or the surface of the water-absorbent resin particle when the water-absorbent resin particle absorbs water in physiological saline, and is expressed as the ratio (%) of the elusion content per 1 g of the water-absorbent resin particle. The dissolution content of the water-absorbent resin particle is preferably 19 mass% or less, more preferably 17 mass% or less, still more preferably 15 mass% or less, and is preferably 0 mass% or more, more preferably 5 mass%. The preferable range is 0 to 19 mass%, 5 to 15 mass%, or the like.

The dissolution content of the water-absorbent resin particle is a value measured by the method described in Examples.

The water-absorbent resin is usually in the form of a particle. The median particle diameter of the water-absorbent resin particle is preferably 150 μm or more, 200 μm or more, 250 μm or more, 280 μm or more, 300 μm or more, or 350 μm or more from the viewpoint of suitably exerting the effect of the present invention while avoiding local absorption in an absorbent article. In addition, the median particle diameter is preferably 850 μm or less, 600 μm or less, 550 μm or less, 500 μm or less, 450 μm or less, or 400 μm or less from the viewpoint of suitably exerting the effect of the present invention while obtaining a comfortable touch in an absorbent article. That is, the median particle diameter is preferably 150 to 850 μm, more preferably 200 to 600 μm, still more preferably 250 to 500 μm, still more preferably 300 to 450 μm, and still more preferably 320 to 400 μm. Also in the water-absorbent resin composition of the present invention, the median particle diameter is preferably 150 to 850 μm, more preferably 200 to 600 μm, further preferably 250 to 500 μm, further preferably 300 to 450 μm, and further more preferably 320 to 400 μm.

The water-absorbent resin particle may be in a form in which fine particles (primary particles) are aggregated (secondary particles) in addition to a form in which each particle is a single particle. Examples of the shape of the primary particle include a substantially spherical shape, an indefinite crushed shape, and a plate shape. When the primary particle is produced by reversed phase suspension polymerization, examples thereof include single particle shapes such as a perfect spherical shape or a substantially spherical shape having a smooth surface shape such as an elliptical spherical shape.

The median particle diameter of the water-absorbent resin particle can be measured using a JIS standard sieve. Specifically, the median particle diameter is a value measured by the method described in Examples.

As the polymerization method of the water-soluble ethylenically unsaturated monomer, an aqueous solution polymerization method, an emulsion polymerization method, a reversed phase suspension polymerization method, and the like, which are representative polymerization methods, are used. In the aqueous solution polymerization method, polymerization is performed by heating a water-soluble ethylenically unsaturated monomer aqueous solution, while stirring the aqueous solution as necessary. In the reversed phase suspension polymerization method, polymerization is performed by heating a water-soluble ethylenically unsaturated monomer in a hydrocarbon dispersion medium under stirring.

An example of the method for producing the water-absorbent resin particle will be described below.

Specific examples of the method for producing the water-absorbent resin particle include: a method in which a water-soluble ethylenically unsaturated monomer is subjected to reversed phase suspension polymerization in a hydrocarbon dispersion medium to produce the water-absorbent resin particle, the method including a step of performing polymerization in the presence of a radical polymerization initiator; and a step of surface-crosslinking the hydrous gel obtained in the polymerization in the presence of a surface-crosslinking agent. In the method for producing the water-absorbent resin particle of the present invention, an internal crosslinking agent may be added to the water-soluble ethylenically unsaturated monomer as necessary to form a hydrous gel having an internal crosslinked structure.

<Polymerization Step>

[Water-Soluble Ethylenically Unsaturated Monomer]

Examples of the water-soluble ethylenically unsaturated monomer include: (meth) acrylic acid (In the present specification, "acrylic" and "methacrylic" are collectively referred to as "(meth) acrylic". The same applies hereinafter.) and salts thereof; 2-(meth) acrylamide-2-methylpropane sulfonic acid and salts thereof; nonionic monomers such as (meth) acrylamide, N,N-dimethyl (meth) acrylamide, 2-hydroxyethyl (meth) acrylate, N-methylol (meth) acrylamide, and polyethylene glycol mono (meth) acrylate; and amino group-containing unsaturated monomers such as N,N-diethylaminoethyl (meth) acrylate, N,N-diethylaminopropyl (meth) acrylate, and diethylaminopropyl (meth) acrylamide, and quaternized products thereof. Among these water-soluble ethylenically unsaturated monomers, (meth) acrylic acid or a salt thereof, (meth) acrylamide, and N,N-dimethylacrylamide are preferable, and (meth) acrylic acid and a salt thereof are more preferable from the viewpoint of industrial availability and the like. These water-soluble ethylenically unsaturated monomers may be used singly or in combination of two or more kinds thereof.

Among them, acrylic acid and salts thereof are widely used as raw materials of the water-absorbent resin particle, and acrylic acid and/or salts thereof may be copolymerized with other water-soluble ethylenically unsaturated monomers described above. In this case, acrylic acid and/or a salt thereof is preferably used as a main water-soluble ethylenically unsaturated monomer in an amount of 70 to 100 mol% with respect to the total amount of water-soluble ethylenically unsaturated monomers.

The water-soluble ethylenically unsaturated monomer in a state of an aqueous solution may be dispersed in a hydrocarbon dispersion medium to be subjected to reversed phase suspension polymerization. The water-soluble ethylenically unsaturated monomer in the form of an aqueous solution can increase the dispersion efficiency in a hydrocarbon dispersion medium. The concentration of the water-soluble ethylenically unsaturated monomer in this aqueous solution is preferably in a range of 20 mass% to the saturated concentration or less. The concentration of the water-soluble ethylenically unsaturated monomer is more preferably 55 mass% or less, still more preferably 50 mass% or less, and still more preferably 45 mass% or less. On the other hand, the concentration of the water-soluble ethylenically unsaturated monomer is more preferably 25 mass% or more, still more preferably 30 mass% or more, and still more preferably 35 mass% or more.

When the water-soluble ethylenically unsaturated monomer has an acid group such as (meth) acrylic acid or 2-(meth) acrylamide-2-methylpropane sulfonic acid, those in which the acid group has been neutralized in advance with an alkaline neutralizing agent may be used as necessary. Examples of such an alkaline neutralizing agent include: alkali metal salts such as sodium hydroxide, sodium carbonate, sodium hydrogen carbonate, potassium hydroxide, and potassium carbonate; and ammonia. In addition, these alkaline neutralizing agents may be used in the form of an aqueous solution in order to simplify the neutralization operation. The alkaline neutralizing agent described above may be used alone, or may be used in combination of two or more thereof.

The neutralization degree of the water-soluble ethylenically unsaturated monomer with the alkaline neutralizing agent is, as the neutralization degree with respect to all the acid groups of the water-soluble ethylenically unsaturated monomer, preferably 10 to 100 mol%, more preferably 30 to 90 mol%, still more preferably 50 to 85 mol%, and still more preferably 70 to 80 mol%.

[Radical Polymerization Initiator]

Examples of the radical polymerization initiator added in the polymerization step include: persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis (2-amidinopropane) 2 hydrochloride, 2,2'-azobis [2-(N-phenylamidino) propane] 2 hydrochloride, 2,2'-azobis [2-(N-allylamidino) propane] 2 hydrochloride, 2,2'-azobis {2-[1-(2-hydroxyethyl)-2-imidazoline-2-yl] propane} 2 hydrochloride, 2,2'-azobis {2-methyl-N-[1,1-bis (hydroxymethyl)-2-hydroxyethyl] propionamide}, 2,2'-azobis [2-methyl-N-(2-hydroxyethyl)-propionamide], and 4,4'-azobis (4-cyanovaleric acid). Among these radical polymerization initiators, potassium persulfate, ammonium persulfate, sodium persulfate, and 2,2'-azobis (2-amidinopropane) 2 hydrochloride are preferable from the viewpoint of easy availability and easy handling. These radical polymerization initiators may be used alone or in combination of two or more. The radical polymerization initiator can also be used as a redox polymerization initiator in combination with a reducing agent such as sodium sulfite, sodium hydrogen sulfite, ferrous sulfate, and L-ascorbic acid.

The amount of the radical polymerization initiator used is, for example, 0.00005 to 0.01 mol with respect to 1 mol of the water-soluble ethylenically unsaturated monomer. By satisfying such a use amount, a rapid polymerization reaction can be avoided, and the polymerization reaction can be completed in an appropriate time.

[Internal Crosslinking Agent]

The internal crosslinking agent includes those capable of crosslinking a polymer of a water-soluble ethylenically unsaturated monomer to be used. Examples thereof include unsaturated polyesters obtained by reacting polyols such as diols and triols such as (poly) ethylene glycol ["(poly)" means having or not having a "poly" prefix. The same applies hereinafter.], (poly) propylene glycol, 1,4-butanediol, 1,6-hexanediol, trimethylolpropane, and (poly) glycerin with an unsaturated acid such as (meth) acrylic acid, maleic acid, and fumaric acid; bisacrylamides such as N,N-methylenebisacrylamide; di (meth) acrylic acid esters or tri (meth) acrylic acid esters obtained by reacting polyepoxide with (meth) acrylic acid; di (meth) acrylic acid carbamyl esters obtained by reacting polyisocyanate such as tolylene diisocyanate and hexamethylene diisocyanate with hydroxyethyl (meth) acrylate; compounds having two or more polymerizable unsaturated groups, such as allylated starch, allylated cellulose, diallyl phthalate, N,N',N"-triallyl isocyanurate, and divinylbenzene; polyglycidyl compounds such as diglycidyl compounds and triglycidyl compounds such as (poly) ethylene glycol diglycidyl ether, (poly) propylene glycol diglycidyl ether, and (poly) glycerin diglycidyl ether; epihalohydrin compounds such as epichlorohydrin, epibromhydrin, and $\alpha$-methylepichlorohydrin; compounds having two or more reactive functional groups such as isocyanate compounds such as 2,4-tolylene diisocyanate and hexamethylene diisocyanate; and oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol. Among these internal crosslinking agents, a polyglycidyl compound is preferably used, a diglycidyl ether compound is more preferably used, and (poly) ethylene glycol diglycidyl ether, (poly) propylene glycol diglycidyl ether, and (poly) glycerin diglycidyl ether are preferably used. These internal crosslinking agents may be used alone, or may be used in combination of two or more thereof.

The amount of the internal crosslinking agent used is preferably 0.000001 to 0.02 mol, more preferably 0.00001 to 0.01 mol, still more preferably 0.00001 to 0.005 mol, and still more preferably 0.00005 to 0.002 mol, with respect to 1 mol of the water-soluble ethylenically unsaturated monomer.

[Hydrocarbon Dispersion Medium]

Examples of the hydrocarbon dispersion medium include: aliphatic hydrocarbons having 6 to 8 carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion mediums, n-hexane, n-heptane, and cyclohexane are suitably used because they are industrially easily available, have stable quality, and are inexpensive. These hydrocarbon dispersion mediums may be used singly or in combination of two or more kinds thereof. As an example of the mixture of the hydrocarbon dispersion mediums, commercially available products such as Exxsol Heptane (manufactured by Exxon Mobil Corporation: containing heptane and isomer hydrocarbons thereof in 75 to 85 mass%) can also be used to obtain a suitable result.

The amount of the hydrocarbon dispersion medium used is preferably 100 to 1500 parts by mass, and more preferably 200 to 1400 parts by mass with respect to 100 parts by mass of the water-soluble ethylenically unsaturated monomer in the first-stage from the viewpoint of uniformly dispersing the water-soluble ethylenically unsaturated monomer and easily controlling the polymerization temperature. As will be described later, the reversed phase suspension polymerization is performed in one stage (single-stage) or in multi-stages of two or more stages, and the above-described polymerization in the first-stage means the first-stage polymerization reaction in a single-stage polymerization or in a multi-stage polymerization (The same applies hereinafter).

[Dispersion Stabilizer]

(Surfactant)

In the reversed phase suspension polymerization, a dispersion stabilizer can also be used in order to improve the dispersion stability of the water-soluble ethylenically unsaturated monomer in the hydrocarbon dispersion medium. As the dispersion stabilizer, a surfactant can be used.

As the surfactant, for example, a sucrose fatty acid ester, a polyglycerin fatty acid ester, a sorbitan fatty acid ester, a polyoxyethylene sorbitan fatty acid ester, a polyoxyethylene glycerin fatty acid ester, a sorbitol fatty acid ester, a polyoxyethylene sorbitol fatty acid ester, a polyoxyethylene alkyl ether, a polyoxyethylene alkyl phenyl ether, a polyoxyethylene castor oil, a polyoxyethylene hydrogenated castor oil, an alkylallyl formaldehyde condensed polyoxyethylene ether, a polyoxyethylene polyoxypropylene block copolymer, a polyoxyethylene polyoxypropyl alkyl ether, a polyethylene glycol fatty acid ester, an alkyl glucoside, an N-alkyl gluconamide, a polyoxyethylene fatty acid amide, a polyoxyethylene alkylamine, a phosphoric acid ester of a polyoxyethylene alkyl ether, a phosphoric acid ester of a polyoxyethylene alkyl allyl ether, and the like can be used. Among these surfactants, it is particularly preferable to use a sorbitan fatty acid ester, a polyglycerin fatty acid ester, or a sucrose fatty acid ester from the viewpoint of dispersion stability of the monomer. These surfactants may be used singly or in combination of two or more kinds thereof.

The amount of the surfactant used is preferably 0.1 to 30 parts by mass, and more preferably 0.3 to 20 parts by mass, with respect to 100 parts by mass of the water-soluble ethylenically unsaturated monomer in the first-stage.

(Polymeric Dispersant)

As the dispersion stabilizer used in the reversed phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymer, maleic anhydride-modified EPDM (ethylene-propylene-diene terpolymer), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, ethyl hydroxyethyl cellulose, and the like. Among these polymeric dispersants, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are particularly preferably used from the viewpoint of dispersion stability of monomers. These polymeric dispersants may be used singly or in combination of two or more kinds thereof.

The amount of the polymeric dispersant used is preferably 0.1 to 30 parts by mass, and more preferably 0.3 to 20 parts by mass, with respect to 100 parts by mass of the water-soluble ethylenically unsaturated monomer in the first-stage.

[Other components]

In the method for producing the water-absorbent resin particle, if desired, other components may be added to an aqueous solution containing a water-soluble ethylenically unsaturated monomer to perform reversed phase suspension polymerization. As other components, various additives such as a thickener and a chain transfer agent can be added.

As an example, the reversed phase suspension polymerization can be performed by adding a thickener to an aqueous solution containing a water-soluble ethylenically unsaturated monomer. By thus adding a thickener to adjust the viscosity of the aqueous solution, the obtained median particle diameter can be controlled in the reversed phase suspension polymerization.

As the thickener, for example, hydroxyethyl cellulose, hydroxypropyl cellulose, methyl cellulose, carboxymethyl cellulose, polyacrylic acid, a (partial) neutralized polyacrylic acid, polyethylene glycol, polyacrylamide, polyethyleneimine, dextrin, sodium alginate, polyvinyl alcohol, polyvinyl pyrrolidone, polyethylene oxide, or the like can be used. When the stirring rate during polymerization is the same, the primary particle and/or the secondary particle of the obtained particles tend to be larger as the aqueous solution of the water-soluble ethylenically unsaturated monomer has a higher viscosity.

[Reversed Phase Suspension Polymerization]

In performing the reversed phase suspension polymerization, for example, a monomer aqueous solution containing a water-soluble ethylenically unsaturated monomer is dispersed in a hydrocarbon dispersion medium in the presence of a dispersion stabilizer. As long as it is before the polymerization reaction is started, the dispersion stabilizer (surfactant or polymeric dispersant) may be added before or after the monomer aqueous solution is added.

Among them, from the viewpoint of easily reducing the amount of the hydrocarbon dispersion medium remaining in the obtained water-absorbent resin particle, it is preferable to disperse the monomer aqueous solution in the hydrocarbon dispersion medium in which the polymeric dispersant has been dispersed, then further disperse the surfactant, and then perform polymerization.

Such reversed phase suspension polymerization can be performed in one stage or in multi-stages of two or more stages. In addition, from the viewpoint of enhancing productivity, 2 to 3 stages are preferably performed.

When the reversed phase suspension polymerization is performed in multi-stages of two or more stages, the first-stage reversed phase suspension polymerization may be performed, and then a water-soluble ethylenically unsaturated monomer may be added to and mixed with the reaction mixture obtained in the first-stage polymerization reaction to perform reversed phase suspension polymerization in the second-stage or the later stages in the same manner as in the first-stage. In the reversed phase suspension polymerization in each of the second-stage and the later stages, in addition to the water-soluble ethylenically unsaturated monomer, a radical polymerization initiator is preferably added to perform reversed phase suspension polymerization within the range of the molar ratio of each component with respect to the water-soluble ethylenically unsaturated monomer described above, based on the amount of the water-soluble ethylenically unsaturated monomer added in the reversed phase suspension polymerization in each of the second-stage or the later stages. Also in the polymerization in the second-stage or the later stages, the internal crosslinking agent may be added to the water-soluble ethylenically unsaturated monomer, as necessary.

The reaction temperature of the polymerization reaction is preferably 20 to 110°C and more preferably 40 to 90°C from the viewpoint of rapidly progressing the polymerization and shortening the polymerization time to enhance the economic efficiency, and easily removing the polymerization heat to smoothly perform the reaction.

<Surface-Crosslinking Step>

Next, the water-absorbent resin particle of the present invention is obtained by adding a surface-crosslinking agent into, and crosslinking the hydrous gel having an internal crosslinked structure obtained in the polymerization of the water-soluble ethylenically unsaturated monomer (surface-crosslinking reaction). The surface-crosslinking reaction is preferably performed in the presence of the surface-crosslinking agent after the polymerization of the water-soluble ethylenically unsaturated monomer. As described above, after the polymerization, the hydrous gel having an internal crosslinked structure is subjected to a surface-crosslinking reaction. Thereby, it is possible to obtain a water-absorbent resin particle having a high crosslinking density in the vicinity of the surface of the water-absorbent resin particle and having enhanced various performances such as water absorption capacity under a load.

Examples of the surface-crosslinking agent include compounds having two or more reactive functional groups. Examples thereof include: polyols such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds such as (poly) ethylene glycol diglycidyl ether, (poly) glycerin diglycidyl ether, (poly) glycerin triglycidyl ether, trimethylolpropane triglycidyl ether, (poly) propylene glycol polyglycidyl ether, and (poly) glycerol polyglycidyl ether; haloepoxy compounds such as epichlorohydrin, epibromhydrin, and $\alpha$-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylene bisoxazoline; carbonate compounds (for example, alkylene carbonates) such as ethylene carbonate, propylene carbonate, 4,5-dimethyl-1,3-dioxolane-2-one, 4,4-dimethyl-1,3-dioxolane-2-one, 4-ethyl-1,3-dioxolane-2-one, 4-hydroxymethyl-1,3-dioxolane-2-one, 1,3-dioxane-2-one, 4-methyl-1,3-dioxane-2-one, 4,6-dimethyl-1,3-dioxane-2-one, and 1,3-dioxolpane-2-one; hydroxyalkylamide compounds such as bis [N,N-di (β-hydroxyethyl)] adipamide. Among these surface-crosslinking agents, polyglycidyl compounds such as (poly) ethylene glycol diglycidyl ether, (poly) glycerin diglycidyl ether, (poly) glycerin triglycidyl ether, trimethylolpropane triglycidyl ether, (poly) propylene glycol polyglycidyl ether, and (poly) glycerol polyglycidyl ether; and carbonate compounds (for example, alkylene carbonates) such as ethylene carbonate, propylene carbonate, 4,5-dimethyl-1,3-dioxolane-2-one, 4,4-dimethyl-1,3-dioxolane-2-one, 4-ethyl-1,3-dioxolane-2-one, 4-hydroxymethyl-1,3-dioxolane-2-one, 1,3-dioxane-2-one, 4-methyl-1,3-dioxane-2-one, 4,6-dimethyl-1,3-dioxane-2-one, and 1,3-dioxopane-2-one are preferable. These surface-crosslinking agents may be used alone, or may be used in combination of two or more thereof.

The amount of the surface-crosslinking agent used is preferably 0.00001 to 0.01 mol, more preferably 0.00005 to 0.005 mol, and still more preferably 0.0001 to 0.002 mol, with respect to 1 mol of the total amount of the water-soluble ethylenically unsaturated monomers used for polymerization.

As a method for adding the surface-crosslinking agent, the surface-crosslinking agent may be added as it is, may be added as an aqueous solution, or may be added as a solution using a hydrophilic organic solvent as a solvent as necessary. Examples of the hydrophilic organic solvent include: lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used alone, or may be used in combination of two or more thereof, or as a mixed solvent with water.

The surface-crosslinking agent only needs to be added after the polymerization reaction of the water-soluble ethylenically unsaturated monomer is almost completed. The surface-crosslinking agent is preferably added in the presence of moisture in the range of 1 to 400 parts by mass, more preferably in the presence of moisture in the range of 5 to 200 parts by mass, still more preferably in the presence of moisture in the range of 10 to 100 parts by mass, and still more preferably in the presence of moisture in the range of 20 to 60 parts by mass, with respect to 100 parts by mass of the water-soluble ethylenically unsaturated monomer. The amount of moisture means the total amount of moisture contained in the reaction system and moisture used as necessary when the surface-crosslinking agent is added.

The reaction temperature in the surface-crosslinking reaction is preferably 50 to 250°C, more preferably 60 to 180°C, still more preferably 60 to 140°C, and still more preferably 70 to 120°C. The reaction time of the surface-crosslinking reaction is preferably 1 to 300 minutes, and more preferably 5 to 200 minutes.

<Drying Step>

The method may include a drying step of removing water, a hydrocarbon dispersion medium, and the like through distillation by externally applying energy such as heat after performing the reversed phase suspension polymerization described above. When the hydrous gel is dehydrated after the reversed phase suspension polymerization, the system in which the hydrous gel is dispersed in the hydrocarbon dispersion medium is heated to once distill water and the hydrocarbon dispersion medium out of the system through azeotropic distillation. If only the distilled hydrocarbon dispersion medium is returned to the system, azeotropic distillation can be continuously performed. In that case, the temperature in the system during drying is maintained at the azeotropic temperature with the hydrocarbon dispersion medium or lower, which is preferable from the viewpoint that the resin is hardly deteriorated. Subsequently, water and the hydrocarbon dispersion medium are distilled off to obtain a water-absorbent resin particle. The treatment conditions in the drying step after the polymerization is controlled to adjust the amount of removed water. Thus, the various performances of the obtained water-absorbent resin particle can be controlled.

In the drying step, the drying treatment by distillation may be performed under normal pressure or under reduced pressure. From the viewpoint of enhancing the drying efficiency, the drying treatment may be performed under a flow of nitrogen or the like. When the drying treatment is performed under normal pressure, the drying temperature is preferably 70 to 250°C, more preferably 80 to 180°C, still more preferably 80 to 140°C, and still more preferably 90 to 130°C. When the drying treatment is performed under reduced pressure, the drying temperature is preferably 40 to 160°C, and more preferably 50 to 110°C.

The water content of the water-absorbent resin particle obtained by the production method of the present invention is preferably 20 mass% or less, more preferably 15 mass% or less, still more preferably 10 mass% or less. The preferable range is 1 to 10 mass% or the like.

When the surface-crosslinking step is performed with a surface-crosslinking agent after monomers are polymerized through reversed phase suspension polymerization, the drying step is performed through the above-described distillation after the surface-crosslinking step is completed. Alternatively, the surface-crosslinking step and the drying step may be performed simultaneously.

The water-absorbent resin composition of the present invention may contain an additive according to the purpose. Examples of such additives include inorganic powders, surfactants, oxidants, reducing agents, radical chain inhibitors, antioxidants, and antibacterial agents. For example, an amorphous silica is added as the inorganic powder in an amount of 0.05 to 5 parts by mass with respect to 100 parts by mass of the water-absorbent resin particle, thereby making it possible to further improve the fluidity of the water-absorbent resin composition. The additive is preferably hydrophilic or water-soluble.

In the water-absorbent resin composition of the present invention, the content of the water-absorbent resin particle (excluding additives) is preferably 70 mass% or more, more preferably 80 mass% or more, and still more preferably 90 mass% or more.

The physiological saline-retention amount of the water-absorbent resin composition obtained by the production method of the present invention is preferably 25 g/g or more, more preferably 30 g/g or more, and is preferably 60 g/g or less, more preferably 55 g/g or less, still more preferably 50 g/g or less. The preferable range is 25 to 60 g/g, 30 to 50 g/g, and the like.

The physiological saline-absorption amount under a load of 4.14 kPa of the water-absorbent resin composition obtained by the production method of the present invention is preferably 5 ml/g or more, more preferably 10 ml/g or more, still more preferably 15 ml/g or more, and is preferably 40 ml/g or less, more preferably 35 ml/g or less, still more preferably 30 ml/g or less. The preferable range is 5 to 40 ml/g, 10 to 30 ml/g, or the like.

Each of the physiological saline-retention amount and the physiological saline-absorption amount under a load of 4.14 kPa of the water-absorbent resin composition is a value measured by the method described in Examples.

From the viewpoint of more suitably exerting the effect of the present invention, the water absorption rate of the water-absorbent resin composition by the Vortex method is preferably 60 seconds or less, more preferably 50 seconds or less, and still more preferably 40 seconds or less, and is preferably 1 second or more, more preferably 3 seconds or more, and still more preferably 10 seconds or more. The preferable range is 1 to 60 seconds, 3 to 40 seconds, and the like.

The water absorption rate of the water-absorbent resin composition by the Vortex method is a value measured by the method described in Examples.

The dissolution content of the water-absorbent resin composition obtained by the production method of the present invention is preferably 19 mass% or less, more preferably 17 mass% or less, still more preferably 15 mass% or less, and is preferably 0 mass% or more, more preferably 5 mass% or more. The preferable range is 0 to 19 mass%, 5 to 15 mass%, or the like.

The dissolution content of the water-absorbent resin composition is a value measured by the method described in Examples.

The water-absorbent resin composition of the present invention has a "yellowness" of preferably 39 or less, more preferably 36 or less, and still more preferably 33 or less as evaluated by the method described in Examples. The lower limit of the yellowness is, for example, 5.

The water-absorbent resin composition of the present invention has an "initial value of gel strength" of preferably 5,000 N/m$^2$ or more, more preferably 6,000 N/m$^2$ or more, still more preferably 6,500 N/m$^2$ or more as evaluated by the method described in Examples. The upper limit of the initial value of gel strength is, for example, 20,000 N/m$^2$.

The water-absorbent resin composition of the present invention has a "gel strength after standing at 37°C for 28 hours" of preferably 5,000 N/m$^2$ or more, more preferably 6,000 N/m$^2$ or more as evaluated by the method described in Examples. The upper limit of the gel strength is, for example, 20,000 N/m$^2$.

2. Absorbent Material and Absorbent article

The water-absorbent resin composition of the present invention constitutes an absorbent material used for hygienic materials such as sanitary products and disposable diapers, and is suitably used for an absorbent article including the absorbent material.

The absorbent material using the water-absorbent resin composition of the present invention contains the water-absorbent resin composition of the present invention. The absorbent material may further include hydrophilic fibers. Examples of the configuration of the absorbent material include: a sheet-like structure in which the water-absorbent resin composition is fixed on a nonwoven fabric or between a plurality of nonwoven fabrics; a mixed dispersion obtained by mixing the water-absorbent resin composition and hydrophilic fibers so as to have a uniform composition; a sandwich structure in which the water-absorbent resin composition is sandwiched between layered hydrophilic fibers; and a structure in which the water-absorbent resin composition and hydrophilic fibers are wrapped with tissue. The absorbent material may contain other components, for example, an adhesive binder such as a heat-sealable synthetic fiber, a hot melt adhesive, or an adhesive emulsion for enhancing the shape retention of the absorbent material.

The content of the water-absorbent resin composition in the absorbent material is preferably 5 to 100 mass%, more preferably 10 to 95 mass%, still more preferably 20 to 90 mass%, and still more preferably 30 to 80 mass%.

Examples of the hydrophilic fibers include: cellulose fibers such as fluff pulp, mechanical pulp, chemical pulp, and semi-chemical pulp, each of which is obtained from wood; artificial cellulose fibers such as rayon and acetate; and fibers made of synthetic resins such as polyamide, polyester, and polyolefin each of which is hydrophilized. The average fiber length of the hydrophilic fibers is usually 0.1 to 10 mm or may be 0.5 to 5 mm.

The absorbent material using the water-absorbent resin composition of the present invention can be held between a liquid-permeable sheet (top sheet) through which a liquid can pass and a liquid-impermeable sheet (back sheet) through which a liquid cannot pass to form the absorbent article of the present invention. The liquid-permeable sheet is disposed on the side in contact with the body, and the liquid-impermeable sheet is disposed on the opposite side of the side in contact with the body.

Examples of the liquid-permeable sheet include: nonwoven fabrics such as an air-through type, a spunbond type, a chemical bond type, and a needle punch type made of fibers such as polyethylene, polypropylene, and polyester; and porous synthetic resin sheets. Examples of the liquid-impermeable sheet include synthetic resin films made of resins such as polyethylene, polypropylene, and polyvinyl chloride.

EXAMPLES

Hereinafter, the present invention will be described in detail with reference to Examples and Comparative Examples. However, the present invention is not limited to Examples.

The water-absorbent resin particles obtained in the following Production Examples and the water-absorbent resin compositions obtained in Examples and Comparative Examples were evaluated in the following various tests. Unless otherwise specified, the measurement was performed under an environment of a temperature of $25 \pm 2°C$ and a humidity of $50 \pm 10\%$.

[Production of Water-Absorbent Resin Particle]

<Production Example 1>

Prepared was a 2 L round-bottomed cylindrical separable flask having an inner diameter of 11 cm and equipped with a reflux condenser, a dropping funnel, a nitrogen gas introduction tube, and a stirring blade having two stages of 4-blade inclined paddle blades having a blade diameter of 5 cm as a stirrer. To this flask, 293 g of n-heptane as a hydrocarbon dispersion medium and 0.736 g of a maleic anhydride-modified ethylene-propylene copolymer (Mitsui Chemicals, Inc.; Hi-WAX 1105A) as a polymeric dispersant were added, and the temperature was raised to 80°C with stirring to dissolve the dispersant, then the mixture was cooled to 50°C. On the other hand, 92.0 g (1.03 mol) of a 80.5 mass% aqueous solution of acrylic acid as a water-soluble ethylenically unsaturated monomer was placed in a beaker having an internal volume of 300 mL, 147.7 g of a 20.9 mass% aqueous solution of sodium hydroxide was added dropwise while cooling with ice water to perform neutralization at 75 mol%, and then 0.092 g of hydroxyl-ethyl cellulose (Sumitomo Seika Chemicals Company, Limited.; HEC AW-15F) as a thickener, 0.0736 g (0.272 mmol) of potassium persulfate as a water-soluble radical polymerization agent, and 0.010 g (0.057 mmol) of ethylene glycol diglycidyl ether as an internal crosslinking agent were added thereto and dissolved, thereby preparing a first-stage aqueous solution. Then, the aqueous solution prepared above was added to a separable flask and stirred for 10 minutes, and then further added thereto was a surfactant solution: in a 20 mL-vial, 0.736 g of a sucrose stearate (RYOTO Sugar Ester S-370 manufactured by MITSUBISHI KAGAKU FOODS) having HLB3 as a surfactant was dissolved by heating in 6.62 g of n-heptane. The mixture was stirred with a stirrer at a rotation speed of 550 rpm while the system was sufficiently replaced with nitrogen. Thereafter, the flask was immersed in a water bath at 70°C and heated, and polymerization was performed for 60 minutes to obtain a first-stage polymerization slurry liquid.

On the other hand, 128.8 g (1.43 mol) of a 80.5 mass% aqueous solution of acrylic acid as a water-soluble ethylenically unsaturated monomer was placed in another beaker having an internal volume of 500 mL, and 159.0 g of a 27 mass% aqueous solution of sodium hydroxide was added dropwise while cooling with ice water to perform neutralization at 75 mol%. Then, 0.103 g (0.381 mmol) of potassium persulfate as a water-soluble radical polymerization initiator and 0.0116 g (0.067 mmol) of ethylene glycol diglycidyl ether as an internal crosslinking agent were added thereto and dissolved, thereby preparing a second-stage aqueous solution.

The system of the separable flask was cooled to 27°C while being stirred with a stirrer at a rotation speed of 1000 rpm. Then the whole amount of the second-stage aqueous solution was added to the first-stage polymerization slurry liquid, and the system was replaced with nitrogen for 30 minutes. Then, the flask was immersed in a water bath at 70°C again and heated, and the polymerization reaction was performed for 60 minutes to obtain a hydrous gel polymer.

Thereafter, the flask was immersed in an oil bath set at 125°C, and 253.9 g of water was removed to the outside of the system while refluxing n-heptane by azeotropic distillation of n-heptane and water. Thereafter, 4.42 g (0.507 mmol) of a 2 mass% aqueous solution of ethylene glycol diglycidyl ether as a surface-crosslinking agent was added to the flask, and the mixture was held at 83°C for 2 hours.

Thereafter, n-heptane was evaporated at 125°C, and the resultant was dried, and further passed through a sieve with a mesh size of 850 $\mu$m to obtain 217.2 g of a water-absorbent resin particle (1). The water-absorbent resin particle (1) had a physiological saline-retention amount of 42 g/g and a physiological saline-absorption amount of 15 mL/g under a load of 4.14 kPa.

<Production Example 2>

The same operation as those in Production Example 1 was performed except that, in Production Example 1, the amount of water to be removed out of the system by azeotropic distillation of n-heptane and water was changed from 253.9 g to 265.9 g, thereby obtaining 221.9 g of a water-absorbent resin particle (2). The water-absorbent resin particle (2) had a physiological saline-retention amount of 51 g/g and a physiological saline-absorption amount of 13 mL/g under a load of 4.14 kPa.

<Production Example 3>

The same operation as those in Production Example 1 was performed except that, in Production Example 1, the amount of water to be removed out of the system by azeotropic distillation of n-heptane and water was changed from 253.9 g to 240.7 g, thereby obtaining 222.1 g of a water-absorbent resin particle (3). The water-absorbent resin particle (3) had a physiological saline-retention amount of 32 g/g and a physiological saline-absorption amount of 30 mL/g under a load of 4.14 kPa.

[Production of Water-Absorbent Resin Composition]

<Example 1>

In a round-bottomed cylindrical separable flask having an inner diameter of 11 cm and equipped with an anchor type stirring blade made of fluororesin, 20 g of the water-absorbent resin particle (1), which was obtained in Production Example 1, was weighed. Next, while stirring at 300 rpm, 0.2 g of a 20 mass% aqueous solution of sodium sulfite was taken with a 3 mL syringe, the tip of the syringe being provided with Fine Atomizer Oral (Yoshikawa Kasei Co., Ltd.), and sprayed into the separable flask for 1 second. Subsequently, 0.2 g of a 1 mass% aqueous solution of L(+)-ascorbic acid was sprayed in the same manner as described above, and a mixture was obtained after 10-minute stirring. This mixture was heated at 100°C for 30 minutes, and then 0.5 parts by mass of amorphous silica (Oriental Silicas Corporation, Tokusil NP-S) was further mixed with respect to 100 parts by mass of the water-absorbent resin particle (1) to obtain a water-absorbent resin composition (1).

<Example 2>

The same operation as those in Example 1 was performed except that, in Example 1, the order of spraying and adding each aqueous solution was changed from the order of: 0.2 g of the 20 mass% aqueous solution of sodium sulfite, followed by 0.2 g of the 1 mass% aqueous solution of L(+)-ascorbic acid to the order of: 0.2 g of the 1 mass% aqueous solution of L(+)-ascorbic acid, followed by 0.2 g of the 20 mass% aqueous solution of sodium sulfite, thereby obtaining a water-absorbent resin composition (2).

<Example 3>

The same operation as those in Example 1 was performed except that, in Example 1, 0.2 g of the 1 mass% aqueous solution of L(+)-ascorbic acid was changed to 0.2 g of a 0.2 mass% aqueous solution of L(+)-ascorbic acid, thereby obtaining a water-absorbent resin composition (3).

<Example 4>

The same operation as those in Example 1 was performed except that, in Example 1, 0.2 g of the 1 mass% aqueous solution of L(+)-ascorbic acid was changed to 0.5 g of a 0.02 mass% aqueous solution of L(+)-ascorbic acid, thereby obtaining a water-absorbent resin composition (4).

<Example 5>

The same operation as those in Example 1 was performed except that, in Example 1, the addition amount of the 20 mass% aqueous solution of sodium sulfite was changed from 0.2 g to 2 g, and 0.2 g of the 1 mass% aqueous solution of L(+)-ascorbic acid was changed to 0.2 g of a 2 mass% aqueous solution of L(+)-ascorbic acid, thereby obtaining a water-absorbent resin composition (5).

<Example 6>

In a round-bottomed cylindrical separable flask having an inner diameter of 11 cm and equipped with an anchor type stirring blade made of fluororesin, 20 g of the water-absorbent resin particle (1), which was obtained in Production Example 1, was weighed. Next, while stirring at 300 rpm, 2 g of a 20 mass% aqueous solution of sodium sulfite was taken with a 3 mL syringe, the tip of the syringe being provided with Fine Atomizer Oral (Yoshikawa Kasei Co.,Ltd.), and sprayed into the separable flask for 1 second. Subsequently, 0.2 g of a 2 mass% aqueous solution of L(+)-ascorbic acid was sprayed in the same manner as described above, and a mixture was obtained after 10-minute stirring. The mixture was heated at 100°C for 30 minutes to obtain a heated mixture.

Into 100 parts by mass of the heated mixture, 0.7 parts by mass of diethylenetriaminepentacetic acid (DTPA) was added as a powder. The resultant was mixed with a cross rotary mixer manufactured by MEIWA KOGYO for 30 minutes (conditions: revolution speed 50 rpm, rotation speed 50 rpm), and 0.5 parts by mass of amorphous silica (Oriental Silicas Corporation, Tokusil NP-S) was further mixed with respect to 100 parts by mass of the water-absorbent resin particle (1). Thus, a water-absorbent resin composition (6) was obtained.

<Comparative Example 1>

To 100 parts by mass of the water-absorbent resin particle (1), which was obtained in Production Example 1, 0.2 parts by mass of sodium sulfite and 0.01 parts by mass of L(+)-ascorbic acid were added as a powder, and the resultant was mixed with a cross rotary mixer manufactured by MEIWA KOGYO for 30 minutes (conditions: revolution speed 50 rpm, rotation speed 50 rpm), and 0.5 parts by mass of amorphous silica (Oriental Silicas Corporation, Tokusil NP-S) was further mixed with respect to 100 parts by mass of the water-absorbent resin particle (1). Thus, a water-absorbent resin composition (7) was obtained.

<Comparative Example 2>

The same operation as those in Comparative Example 2 was performed except that, in Comparative Example 1, the addition amount of sodium sulfite was changed from 0.2 parts by mass to 0.1 parts by mass, and the addition amount of L(+)-ascorbic acid was changed from 0.01 parts by mass to 0.02 parts by mass, thereby obtaining a water-absorbent resin composition (8).

<Comparative Example 3>

To 100 parts by mass of the water-absorbent resin particle (1), which was obtained in Production Example 1, 2 parts by mass of sodium sulfite, 0.02 parts by mass of L(+)-ascorbic acid, and 0.7 parts by mass of diethylenetriaminepentacetic acid (DTPA) as a powder were added, and the resultant was mixed with a cross rotary mixer manufactured by MEIWA KOGYO for 30 minutes (conditions: revolution speed 50 rpm, rotation speed 50 rpm), and 0.5 parts by mass of amorphous silica (Oriental Silicas Corporation, Tokusil NP-S) was further mixed with respect to 100 parts by mass of the water-absorbent resin particle (1). Thus, a water-absorbent resin composition (9) was obtained.

<Comparative Example 4>

The same operation as those in Example 1 was performed except that, in Example 1, 0.2 g of the 1 mass% aqueous solution of L(+)-ascorbic acid was not used, thereby obtaining a water-absorbent resin composition (10).

<Comparative Example 5>

The same operation as those in Example 1 was performed except that, in Example 1, 0.2 g of the 20 mass% aqueous solution of sodium sulfite was not used, thereby obtaining a water-absorbent resin composition (11).

<Comparative Example 6>

In a round-bottomed cylindrical separable flask having an inner diameter of 11 cm and equipped with an anchor type stirring blade made of fluororesin, 20 g of the water-absorbent resin particle (1), which was obtained in Production Example 1, was weighed. Next, while stirring at 300 rpm, 0.2 g of a 20 mass% aqueous solution of sodium sulfite was taken with a 3 mL syringe, the tip of the syringe being provided with Fine Atomizer Oral (Yoshikawa Kasei Co.,Ltd.), and sprayed into the separable flask for 1 second. Then, a mixture was obtained after 10-minute stirring. The mixture was heated at 100°C for 30 minutes to obtain a heated mixture.

Into 100 parts by mass of the heated mixture, 0.01 parts by mass of L(+)-ascorbic acid was added as a powder. The resultant was mixed with a cross rotary mixer manufactured by MEIWA KOGYO for 30 minutes (conditions: revolution speed 50 rpm, rotation speed 50 rpm), and 0.5 parts by mass of amorphous silica (Oriental Silicas Corporation, Tokusil NP-S) was further mixed with respect to 100 parts by mass of the water-absorbent resin particle (1). Thus, a water-absorbent resin composition (12) was obtained.

<Comparative Example 7>

In a round-bottomed cylindrical separable flask having an inner diameter of 11 cm and equipped with an anchor type stirring blade made of fluororesin, 20 g of the water-absorbent resin particle (1), which was obtained in Production Example 1, was weighed. Next, while stirring at 300 rpm, 0.2 g of a 1 mass% aqueous solution of L(+)-ascorbic acid was taken with a 3 mL syringe, the tip of the syringe being provided with Fine Atomizer Oral (Yoshikawa Kasei Co.,Ltd.), and sprayed into the separable flask for 1 second. Then, a mixture was obtained after 10-minute stirring. The mixture was heated at 100°C for 30 minutes to obtain a heated mixture.

Into 100 parts by mass of the heated mixture, 0.2 parts by mass of sodium sulfite was added as a powder. The resultant was mixed with a cross rotary mixer manufactured by MEIWA KOGYO for 30 minutes (conditions: revolution speed 50 rpm, rotation speed 50 rpm), and 0.5 parts by mass of amorphous silica (Oriental Silicas Corporation, Tokusil NP-S) was further mixed with respect to 100 parts by mass of the water-absorbent resin particle (1). Thus, a water-absorbent resin composition (13) was obtained.

<Example 7>

The same operation as those in Example 1 was performed except that, in Example 1, 20 g of the water-absorbent resin particle (2), which was obtained in Production Example 2, was used in place of 20 g of the water-absorbent resin particle (1), which was obtained in Production Example 1, thereby obtaining a water-absorbent resin composition (14).

<Example 8>

The same operation as those in Example 3 was performed except that, in Example 3, 20 g of the water-absorbent resin particle (2), which was obtained in Production Example 2, was used in place of 20 g of the water-absorbent resin particle (1), which was obtained in Production Example 1, thereby obtaining a water-absorbent resin composition (15).

<Example 9>

The same operation as those in Example 1 was performed except that, in Example 1, 20 g of the water-absorbent resin particle (2), which was obtained in Production Example 2, was used in place of 20 g of the water-absorbent resin particle (1), which was obtained in Production Example 1, and the addition amount of the 20 mass% aqueous solution of sodium sulfite was changed from 0.2 g to 0.1 g, thereby obtaining a water-absorbent resin composition (16).

<Example 10>

The same operation as those in Example 1 was performed except that, in Example 1, 20 g of the water-absorbent resin particle (3), which was obtained in Production Example 3, was used in place of 20 g of the water-absorbent resin particle (1), which was obtained in Production Example 1, thereby obtaining a water-absorbent resin composition (17).

[Evaluation of Water-Absorbent Resin Particle]

<Physiological Saline-Retention Amount>

A cotton bag (cotton broad cloth No. 60, width 100 mm × length 200 mm) in which 2.0 g of the water-absorbent resin particle was weighed was placed in a 500 mL beaker. Into the cotton bag containing the water-absorbent resin particle, 500 g of a 0.9 mass% sodium chloride aqueous solution (physiological saline) was poured at a time so as not to form lumps. Then, the top of the cotton bag was tied with a rubber band, and the cotton bag was allowed to stand for 30 minutes to swell the water-absorbent resin particle. After 30 minutes, the cotton bag was dehydrated for 1 minute using a dehydrator (manufactured by KOKUSAN Co. Ltd.; product number: H-122) set to a centrifugal force of 167 G, and the mass $W_d$ (g) of the dehydrated cotton bag containing the swollen gel was measured. The same operation was performed without adding the water-absorbent resin particle, the empty mass $W_e$ (g) of the swollen cotton bag was measured, and the physiological saline-retention amount was calculated from the following equation.

Physiological saline-retention amount (g/g) = [Wd-We]/2.0

<Physiological Saline-Absorption Amount under Load of 4.14 kPa>

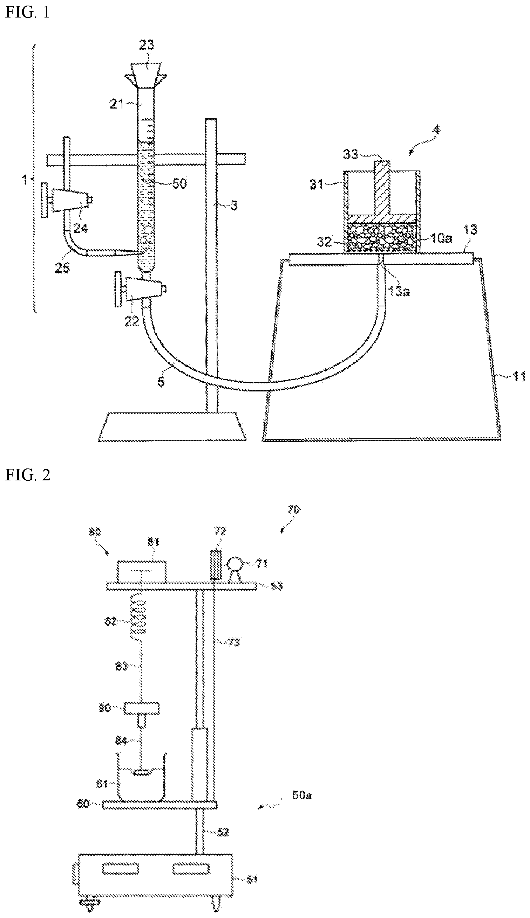

The physiological saline-absorption amount under a load of 4.14 kPa (water absorption under load) was measured using a measuring device schematically shown in Fig. 1. Each water-absorbent resin particle was measured twice, and the average value was obtained. The measuring device includes a burette section 1, a clamp 3, a conduit 5, a stand 11, a measuring table 13, and a measuring section 4 placed on the measuring table 13. The burette section 1 includes a burette pipe 21 with a scale thereon, a rubber stopper 23 to seal the opening at the top of the burette pipe 21, a cock 22 connected to the lower tip of the burette pipe 21, and an air introduction pipe 25 and a cock 24 connected to the lower portion of the burette pipe 21. The burette section 1 is fixed by the clamp 3. The measuring table 13 with a flat plate shape has a through hole 13a with a diameter of 2 mm formed in the central portion thereof, and is supported by the stand 11 having a variable height. The through hole 13a of the measuring table 13 and the cock 22 of the burette section 1 are connected through the conduit 5. The inner diameter of the conduit 5 is 6 mm.

The measuring section 4 includes a cylinder 31 made of PLEXIGLAS, a polyamide mesh 32 bonded to one opening of the cylinder 31, and a weight 33 movable in the vertical direction within the cylinder 31. The cylinder 31 is placed on the measuring table 13 through the polyamide mesh 32. The cylinder 31 has an inner diameter of 20 mm. The polyamide mesh 32 has a mesh size of 75 $\mu$m (200 mesh). The weight 33 has a diameter of 19 mm and a mass of 119.6 g, and can apply a load of 4.14 kPa (0.6 psi) to a water-absorbent resin particle 10a uniformly arranged on the polyamide mesh 32, as described above.

First, the cock 22 and the cock 24 of the burette section 1 were closed, and 0.9 mass% physiological saline adjusted to 25°C was put into the burette pipe 21 through the opening at the top of the burette pipe 21. Next, the opening at the top of the burette pipe 21 was sealed with the rubber stopper 23, and then the cock 22 and the cock 24 were opened. The inside of the conduit 5 was filled with a 0.9 mass% saline 50 such that air bubbles did not contained. The height of the measuring table 13 was adjusted so that the water surface of the 0.9 mass% saline reaching the inside of the through hole 13a was as high as the upper surface of the measuring table 13. After the adjustment, the height of the water surface of the 0.9 mass% saline 50 in the burette pipe 21 was read by the scale of the burette pipe 21, and the position was defined as the zero point (the read value at 0 second).

In the measuring section 4, 0.10 g of the water-absorbent resin particle 10a was uniformly arranged on the polyamide mesh 32 in the cylinder 31, the weight 33 was arranged on the water-absorbent resin particle 10a, and the cylinder 31 was installed so that the central portion thereof coincided with the conduit hole at the central portion of the measuring table 13. The decrease amount of the physiological saline in the burette pipe 21 (that is, the amount of physiological saline absorbed by the water-absorbent resin particle 10a) Wc (mL) was read 60 minutes after the water-absorbent resin particle 10a started to absorb physiological saline from the conduit 5. Then, the physiological saline-absorption capacity of the water-absorbent resin particle 10a under a load of 4.14 kPa was calculated by the following equation.

Physiological saline-absorption capacity under a load of 4.14 kPa (mL/g) = Wc (mL)/mass (g) of the water-absorbent resin particle

<Measurement of water absorption rate by Vortex method>

In a 100 mL beaker, 50 $\pm$ 0.1 g of physiological saline adjusted to a temperature of 25 $\pm$ 0.2°C in a constant temperature water bath was weighed, and stirred with a magnetic stirrer bar (8 mm$\varphi$ $\times$ 30 mm, without a ring) to generate a vortex at a rotation speed of 600 rpm. Into the physiological saline, 2.0 $\pm$ 0.002 g of the water-absorbent resin particle was added at a time. Time (second) was measured from after the water-absorbent resin particle was added until before the vortex on the liquid surface disappeared. The time was defined as the water absorption rate of the water-absorbent resin particle. The water absorption rate is also expressed as Vortex method or vortex time.

<Dissolution content>

In a 500 mL beaker, 500 g of physiological saline was stirred with a stirring bar (cylindrical shape with a diameter of 8 mm $\times$ a length of 30 mm, no ring) rotating at 600 rpm. The physiological saline had a temperature of 25°C. Into the physiological saline, 2.000 g of the water-absorbent particle was added, and a dispersion containing the water-absorbent particle was stirred for three hours. The dispersion was filtered through a 75 $\mu$m standard sieve to recover a filtrate. Eighty grams of the obtained filtrate was weighed in a weighed 100 mL beaker that had been previously subjected to constant weight-determination at 140°C. The filtrate in the beaker was heated in a hot air dryer (FV-320; manufactured by ADVANTEC) at 140°C for 15 hours to remove moisture, and a remaining solid content was measured for mass Wa (g). A blank test was performed by above-described operation without adding the water-absorbent particle into the physiological saline to measure a solid content remaining in the beaker for mass Wb (g). The dissolution content was calculated by an equation below.

$$\text{Dissolution content (mass\%)} = [((Wa-Wb)/80) \times 500/2] \times 100$$

<Median Particle Diameter (Particle Size Distribution)>

Using a sonic vibration type sieving measuring instrument (Robot Sifter RPS-01, manufactured by Seishin Enterprise Co., Ltd.), a JIS standard sieve with mesh sizes of 850 μm, 600 μm, 500 μm, 425 μm, 300 μm, 250 μm, and 180 μm, and a receptacle, 10 g of the water-absorbent resin particle was sieved. The mass of the particles remaining on each sieve was calculated as a mass percentage based on the total amount. The mass percentage of the remaining particles on each sieve was integrated in descending order of particle size. The relationship between the mesh size of sieves and the integrated mass percentage of the remaining particles on each sieve was plotted on a logarithmic probability paper. The plot on the probability paper was connected by a straight line to determine a particle diameter corresponding to the integrated mass percentage of 50 mass%. The particle diameter was determined as the median particle diameter.

<Water Content>

In an aluminum foil case (No. 8) that has been previously subjected to constant weight-determination (W1 (g)), 2.0 g of the water-absorbent resin particle is placed. Then the mouth of the aluminum foil case is lightly closed, and the total mass W2 (g) of the aluminum foil case containing the sample is precisely weighed. The aluminum foil case containing the sample described above is dried for 2 hours in a hot air dryer (manufactured by ADVANTEC; model: FV-320) set at an internal temperature of 105°C. The dried aluminum foil case containing the sample is allowed to cool to room temperature in a desiccator. The total mass W3 (g) of the cooled aluminum foil case containing the sample is measured. The water content of the sample is calculated from the following equation.

$$\text{Water content [mass\%]} = [\{(W2 - W1) - (W3 - W1)\}/(W2 - W1)] \times 100$$

[Evaluation of Water-Absorbent Resin Composition]

<Physiological Saline-Retention Amount>

The physiological saline-retention amount of the water-absorbent resin composition was measured in the same manner as the physiological saline-retention amount measured for the water-absorbent resin particle.

<Physiological Saline-Absorption Amount under Load of 4.14 kPa>

The physiological saline-absorption amount under a load of 4.14 kPa of the water-absorbent resin composition was measured in the same manner as the physiological saline-absorption amount under a load of 4.14 kPa measured for the water-absorbent resin particle.

<Measurement of water absorption rate by Vortex method>

The water absorption rate of the water-absorbent resin composition was measured in the same manner as in the measurement of the water absorption rate by the Vortex method measured for the water-absorbent resin particle.

<Dissolution content>

The dissolution content of the water-absorbent resin composition was measured in the same manner as the dissolution content measured for the water-absorbent resin particle.

<Median Particle Diameter (Particle Size Distribution)>

The median particle diameter (particle size distribution) of the water-absorbent resin composition was measured in the same manner as the median particle diameter (particle size distribution) measured for the water-absorbent resin particle.

<Water Content>

The water content of the water-absorbent resin composition was measured in the same manner as the water content measured for the water-absorbent resin particle.

<Yellow Coloration Test under High Temperature and High Humidity (Measurement of Yellowness)>

In a glass measuring container having an inner diameter of 3 cm, 2.0 g of the water-absorbent resin composition was placed. The yellowness of the water-absorbent resin composition was measured with a colorimeter (Color Meter ZE6000, manufactured by NIPPON DENSHOKU INDUSTRIES CO., LTD.) in which the tristimulus values X, Y, and Z of the colorimeter had been corrected using a standard white plate. From the obtained X, Y, and Z (tristimulus values) of the water-absorbent resin composition, the yellowness was calculated by the following equation, and determined as the initial value.

$$\text{Yellowness} = 100(1.28X - 1.06Z)/Y$$

In addition, the temporal coloring test for the water-absorbent resin composition was performed as follows. That is, 2.0 g of the water-absorbent resin composition was uniformly placed in a glass petri dish having an inner diameter of 3 cm and a depth of 1 cm. The container was stored for 7 days in a thermo-hygrostat (manufactured by ESPEC CORP., LHU-113) set at a temperature of $70 \pm 2°C$ and a relative humidity of $90 \pm 2\%$. Thereafter, the container was taken out from the thermo-hygrostat, left for a while, and cooled to room temperature. The whole amount of the water-absorbent resin composition in the container was placed in a glass measuring container having an inner diameter of 3 cm, and the yellowness of the water-absorbent resin composition was measured with a colorimeter (Color Meter ZE6000, manufactured by NIPPON DENSHOKU INDUSTRIES CO., LTD.). From the obtained X, Y, and Z (tristimulus values) of the water-absorbent resin composition, the yellowness was calculated by the following equation.

$$\text{Yellowness} = 100(1.28X - 1.06Z)/Y$$

<Test of Gel Absorbing Artificial Urine>

The gel (swollen gel), the water-absorbent resin composition that had absorbed water, was tested using artificial urine.

(Preparation of Artificial Urine)

An artificial urine having the following composition was prepared.

Urea: 20.0 g
Sodium chloride: 8.0 g
Calcium chloride dihydrate: 0.3 g
Magnesium sulfate heptahydrate: 0.8 g
L(+)-ascorbic acid: 0.2 g
Ion-exchange water: 970.9 g

(Preparation of Swollen Gel)

In a beaker having an internal volume of 100 mL, 39.0 g of the artificial urine was weighed. Then a magnetic stirrer bar (8 mm$\varphi \times$ 30 mm) was put into the beaker. The beaker was placed on a magnetic stirrer (HS-30D; manufactured by iuchi), and the magnetic stirrer bar was rotated at 600 rpm. Next, 1.00 g of the water-absorbent resin composition was put into the beaker under stirring, and the mixture was stirred until the rotating vortex disappeared and the liquid level became horizontal, thereby preparing a swollen gel as a measurement sample. Immediately after preparing the swollen gel, the beaker containing the swollen gel was covered with wrap (DAIYA WRAP; manufactured by Mitsubishi Chemical Corporation).

(Measurement of Gel Strength)

The gel strength after standing for a predetermined time under each temperature condition was measured using a device having the measurement principle shown in Fig. 2. The device illustrated in Fig. 2 includes a support 50a, a movable base plate 60, a drive unit 70 to drive the movable base plate 60, and a measuring section 80. In the support 50a, a stand 53 is fixed on the top of a support column 52 erected on a support stand 51. The movable base plate 60 is attached to the support column 52 so as to move up and down. A measurement sample (gel) 61 can be mounted on the movable base plate 60. A pulse motor 71 is mounted on the stand 53, and rotates a pulley 72 to move the movable base plate 60 up and down via a wire 73. In the measuring section 80, a disc-attached pressure-sensitive shaft 84 is attached to a load cell 81 to measure distortion caused by deformation via a precision spring 82 and a connecting shaft 83. The disc-attached pressure-sensitive shaft 84 has a disk at the tip. Depending on the measurement conditions, the diameter of the disc can be varied. A weight 90 can be mounted on the top of the disc-attached pressure-sensitive shaft 84. The operating principle of the device to measure gel strength is as follows. The precision spring 82 is fixed to the load cell 81 (stress detector) on the upper side thereof, and connected to the disc-attached pressure-sensitive shaft 84 on the lower side thereof, and vertically suspended with the predetermined weight 90 placed thereon. The movable base plate 60 on which the measurement sample 61 is placed rises at a constant speed by the rotation of the pulse motor 71. A constant speed load is applied to the measurement sample 61 via the precision spring 82, distortion generated by deformation is measured by the load cell 81, and the hardness is measured and calculated. The gel strength value ($N/m^2$) was measured using Curdmeter-MAX (manufactured by ASKA KIKI; product number: ME-500), in the disc-attached pressure-sensitive shaft 84 with a 16 mm$\varphi$ disc, a load of 400 g, a speed of 7 seconds/inch, and the viscous mode setting, and under the conditions for temperature and standing time shown in the (Initial Value of Gel Strength) and (Gel Strength After Standing at 37°C for 28 Hours) below.

(Initial Value of Gel Strength)

The swollen gel was allowed to stand in an environment at a temperature of 25 ± 2°C and a relative humidity of 50 ± 10% for 60 minutes after preparation, and then the gel strength (initial value of gel strength) was measured by the above-described method.

(Gel Strength After Standing at 37°C for 28 Hours)

The swollen gel was allowed to stand in a thermo-hygrostat (manufactured by ESPEC CORP., LHU-113) set at a temperature of 37 ± 2°C and a relative humidity of 60 ± 10% for 28 hours after preparation, and then the gel strength was measured by the above-described method. The value of the gel strength at this time was defined as the gel strength after standing at 37°C for 28 hours.

[Table 1]

| | Water-absorbent resin particle (SAP) | | | | | | | Additive | | | | | | | | | | | Water-absorbent resin composition | | | | | | | Yellowness | | Gel stability evaluation result | |
| | | | | | | | | Additive A | | | Additive B | | | | Addition order of additives A and B | Additive C | | | | | | | | | | | | | |
| | Name | Physiological saline-retention amount [g/g] | Physiological saline-absorption capacity under load of 4.14 kPa [ml/g] | Water absorption rate [sec] | Water content [mass%] | Dissolution content [mass%] | Median particle diameter [μm] | Type | Addition amount (vs. SAP100 parts) [parts by mass] | Addition method | Type | Addition amount (vs. SAP100 parts) [parts by mass] | Addition amount (vs. Additive A100 parts) [parts by mass] | Addition method | | Type | Addition amount (vs. SAP100 parts) [parts by mass] | Addition method | Water-absorbent resin composition | Physiological saline-retention amount [g/g] | Physiological saline-absorption capacity under load of 4.14 kPa [ml/g] | Water absorption rate [sec] | Water content [mass%] | Dissolution content [mass%] | Median particle diameter [μm] | Initial value | After 7 days | Initial value of gel strength [N/m²] | Gel strength after standing for 28 hours [N/m²] |
|---|---|---|---|---|---|---|---|---|---|---|---|---|---|---|---|---|---|---|---|---|---|---|---|---|---|---|---|---|---|---|
| Example 1 | Water-absorbent resin particle (1) | 42 | 15 | 36 | 7 | 10 | 371 | Sodium sulfite | 0.2 | Spraying aqueous solution | L(+)-ascorbic acid | 0.01 | 5 | Spraying aqueous solution | A→B | Absent | - | - | Water-absorbent resin composition (1) | 40 | 15 | 36 | 5 | 9 | 371 | 6 | 31 | 6584 | 6484 |
| Example 2 | Water-absorbent resin particle (1) | 42 | 15 | 36 | 7 | 10 | 371 | Sodium sulfite | 0.2 | Spraying aqueous solution | L(+)-ascorbic acid | 0.01 | 5 | Spraying aqueous solution | B→A | Absent | - | - | Water-absorbent resin composition (2) | 40 | 15 | 36 | 5 | 10 | 370 | 6 | 31 | 6599 | 7205 |
| Example 3 | Water-absorbent resin particle (1) | 42 | 15 | 36 | 7 | 10 | 371 | Sodium sulfite | 0.2 | Spraying aqueous solution | L(+)-ascorbic acid | 0.002 | 1 | Spraying aqueous solution | A→B | Absent | - | - | Water-absorbent resin composition (3) | 42 | 14 | 36 | 4 | 10 | 371 | 7 | 31 | 6547 | 6634 |
| Example 4 | Water-absorbent resin particle (1) | 42 | 15 | 36 | 7 | 10 | 371 | Sodium sulfite | 0.2 | Spraying aqueous solution | L(+)-ascorbic acid | 0.0005 | 0.25 | Spraying aqueous solution | A→B | Absent | - | - | Water-absorbent resin composition (4) | 43 | 15 | 36 | 4 | 10 | 372 | 6 | 31 | 6624 | 7653 |
| Example 5 | Water-absorbent resin particle (1) | 42 | 15 | 36 | 7 | 10 | 371 | Sodium sulfite | 2 | Spraying aqueous solution | L(+)-ascorbic acid | 0.02 | 1 | Spraying aqueous solution | A→B | Absent | - | - | Water-absorbent resin composition (5) | 42 | 15 | 36 | 5 | 10 | 370 | 5 | 32 | 7731 | 7921 |
| Example 6 | Water-absorbent resin particle (1) | 42 | 15 | 36 | 7 | 10 | 371 | Sodium sulfite | 2 | Spraying aqueous solution | L(+)-ascorbic acid | 0.02 | 1 | Spraying aqueous solution | A→B | Diethylenetriaminepentacetic acid | 0.7 | Mixing powder | Water-absorbent resin composition (6) | 42 | 15 | 36 | 4 | 10 | 368 | 5 | 18 | 7373 | 7390 |
| Comparative Example 1 | Water-absorbent resin particle (1) | 42 | 15 | 36 | 7 | 10 | 371 | Sodium sulfite | 0.2 | Mixing powder | L(+)-ascorbic acid | 0.01 | 5 | Mixing powder | - | Absent | - | - | Water-absorbent resin composition (7) | 40 | 15 | 36 | 7 | 12 | 370 | 6 | 40 | 7990 | 7072 |
| Comparative Example 2 | Water-absorbent resin particle (1) | 42 | 15 | 36 | 7 | 10 | 371 | Sodium sulfite | 0.1 | Mixing powder | L(+)-ascorbic acid | 0.02 | 20 | Mixing powder | - | Absent | - | - | Water-absorbent resin composition (8) | 40 | 14 | 36 | 7 | 11 | 370 | 9 | 45 | 7180 | 6707 |
| Comparative Example 3 | Water-absorbent resin particle (1) | 42 | 15 | 36 | 7 | 10 | 371 | Sodium sulfite | 0.2 | Mixing powder | L(+)-ascorbic acid | 0.02 | 1 | Mixing powder | - | Diethylenetriaminepentacetic acid | 0.7 | Mixing powder | Water-absorbent resin composition (9) | 42 | 14 | 36 | 7 | 12 | 373 | 6 | 54 | 5576 | 5712 |
| Comparative Example 4 | Water-absorbent resin particle (1) | 42 | 15 | 36 | 7 | 10 | 371 | Sodium sulfite | 0.2 | Spraying aqueous solution | - | - | - | - | - | Absent | - | - | Water-absorbent resin composition (10) | 45 | 15 | 36 | 5 | 26 | 368 | 6 | 30 | 6584 | 5820 |
| Comparative Example 5 | Water-absorbent resin particle (1) | 42 | 15 | 36 | 7 | 10 | 371 | Absent | - | - | L(+)-ascorbic acid | 0.01 | - | Spraying aqueous solution | - | Absent | - | - | Water-absorbent resin composition (11) | 42 | 13 | 36 | 5 | 11 | 368 | 6 | 31 | 7849 | 0 |
| Comparative Example 6 | Water-absorbent resin particle (1) | 42 | 15 | 36 | 7 | 10 | 371 | Sodium sulfite | 0.2 | Spraying aqueous solution | L(+)-ascorbic acid | 0.01 | 5 | Mixing powder | A→B | Absent | - | - | Water-absorbent resin composition (12) | 42 | 12 | 36 | 5 | 13 | 368 | 6 | 54 | 7731 | 8647 |
| Comparative Example 7 | Water-absorbent resin particle (1) | 42 | 15 | 36 | 7 | 10 | 371 | Sodium sulfite | 0.2 | Mixing powder | L(+)-ascorbic acid | 0.01 | 5 | Spraying aqueous solution | B→A | Absent | - | - | Water-absorbent resin composition (13) | 42 | 11 | 36 | 5 | 14 | 370 | 6 | 51 | 7674 | 7620 |
| Example 7 | Water-absorbent resin particle (2) | 51 | 13 | 33 | 8 | 11 | 372 | Sodium sulfite | 0.2 | Spraying aqueous solution | L(+)-ascorbic acid | 0.01 | 5 | Spraying aqueous solution | A→B | Absent | - | - | Water-absorbent resin composition (14) | 52 | 13 | 33 | 4 | 11 | 372 | 6 | 31 | 13920 | 10203 |
| Example 8 | Water-absorbent resin particle (2) | 51 | 13 | 33 | 8 | 11 | 372 | Sodium sulfite | 0.2 | Spraying aqueous solution | L(+)-ascorbic acid | 0.002 | 1 | Spraying aqueous solution | A→B | Absent | - | - | Water-absorbent resin composition (15) | 50 | 12 | 33 | 4 | 10 | 371 | 7 | 31 | 11645 | 8953 |
| Example 9 | Water-absorbent resin particle (2) | 51 | 13 | 33 | 8 | 11 | 372 | Sodium sulfite | 0.1 | Spraying aqueous solution | L(+)-ascorbic acid | 0.01 | 10 | Spraying aqueous solution | A→B | Absent | - | - | Water-absorbent resin composition (16) | 50 | 13 | 33 | 5 | 11 | 377 | 7 | 31 | 11470 | 7545 |
| Example 10 | Water-absorbent resin particle (3) | 32 | 30 | 39 | 8 | 9 | 370 | Sodium sulfite | 0.2 | Spraying aqueous solution | L(+)-ascorbic acid | 0.01 | 5 | Spraying aqueous solution | A→B | Absent | - | - | Water-absorbent resin composition (17) | 34 | 30 | 39 | 4 | 9 | 370 | 8 | 37 | 17187 | 11363 |

Reference Signs List

1 Burette section
3 Clamp
4 Measuring section
5 Conduit
10a Water-absorbent resin particle
11 Stand
13 Measuring table
13a Through hole
15 Nylon mesh sheet
21 Burette pipe
22 Cock
23 Rubber stopper
24 Cock
25 Air introduction pipe
31 Cylinder
32 Polyamide mesh
33 Weight
50a Support
51 Support column stand
52 Support column
53 Stand
60 Movable base plate
61 Measurement sample
70 Drive unit
71 Pulse motor
72 Pulley
73 Wire
80 Measuring section
81 Load cell
82 Precision spring
83 Connecting shaft
84 Disc-attached pressure-sensitive shaft
90 Weight

**Claims**

1.  A method for producing a water-absorbent resin composition, the method comprising:
    a step of spraying an aqueous solution of a sulfite-based compound, and a step of spraying an aqueous solution of an organic antioxidant, to a water-absorbent resin particle having been subjected to a surface-crosslinking process.

2.  The method for producing a water-absorbent resin composition according to claim 1, wherein the sulfite-based compound includes at least one selected from a group consisting of sodium sulfite, potassium sulfite, calcium sulfite, sodium hydrogen sulfite, potassium hydrogen sulfite, ammonium hydrogen sulfite, sodium metabisulfite, and potassium metabisulfite.

3.  The method for producing a water-absorbent resin composition according to claim 1 or 2, wherein the organic antioxidant includes at least one selected from a group consisting of ascorbic acids, erythorbic acids, gallic acids, protocatechuic acids, benzimidazoles, and alkylhydroxyanisoles.

4.  The method for producing a water-absorbent resin composition according to claim 1 or 2, wherein the water-absorbent resin particle has a dissolution content of 19 parts by mass or less, the dissolution content being determined by a measurement method below.
    (Dissolution content)

In a 500 mL beaker, 500 g of physiological saline is stirred with a stirring bar rotating at 600 rpm. The physiological saline has a temperature of 25°C. Into the physiological saline, 2.000 g of the water-absorbent resin particle is added, and a dispersion containing the water-absorbent resin particle is stirred for three hours. The dispersion is filtered through a 75 μm standard sieve to recover a filtrate. Eighty grams of the obtained filtrate is weighed in a weighed 100 mL beaker that has been previously subjected to constant weight-determination at 140°C. The filtrate in the beaker is heated in a hot air dryer at 140°C for 15 hours to remove moisture, and a remaining solid content is measured for mass Wa (g). A blank test is performed by above-described operation without adding the water-absorbent resin particle into the physiological saline to measure a solid content remaining in the beaker for mass Wb (g). The dissolution content is calculated by an equation below.

$$\text{Dissolution content (mass\%)} = [((Wa-Wb)/80) \times 500/2] \times 100$$

5. The method for producing a water-absorbent resin composition according to claim 1 or 2, wherein the sulfite-based compound is sprayed in an amount of 0.01 parts by mass or more and 3 parts by mass or less with respect to 100 parts by mass of the water-absorbent resin particle.

6. The method for producing a water-absorbent resin composition according to claim 1 or 2, wherein the organic antioxidant is sprayed in an amount of 0.1 parts by mass or more and 20 parts by mass or less with respect to 100 parts by mass of the sulfite-based compound.

7. The method for producing a water-absorbent resin composition according to claim 1 or 2, wherein the water-absorbent resin particle has a physiological saline-retention amount of 25 g/g or more and 60 g/g or less.

8. The method for producing a water-absorbent resin composition according to claim 1 or 2, wherein the water-absorbent resin particle has a physiological saline-absorption amount of 5 ml/g or more and 40 ml/g or less under a load of 4.14 kPa.

FIG. 1

FIG. 2

<table>
<tr><td colspan="2" align="center">INTERNATIONAL SEARCH REPORT</td><td>International application No.<br><br>**PCT/JP2023/012460**</td></tr>
</table>

**A. CLASSIFICATION OF SUBJECT MATTER**

*C08J 3/12*(2006.01)i; *C08J 3/24*(2006.01)i; *C08K 5/00*(2006.01)i; *C08L 101/14*(2006.01)i; *A61F 13/53*(2006.01)i; *C08K 3/30*(2006.01)i; *A61L 15/24*(2006.01)i; *A61L 15/60*(2006.01)i
FI:  C08J3/24 Z; C08L101/14; C08K3/30; C08K5/00; A61F13/53 300; A61L15/24; A61L15/60; C08J3/12 Z CEY

According to International Patent Classification (IPC) or to both national classification and IPC

**B. FIELDS SEARCHED**

Minimum documentation searched (classification system followed by classification symbols)

C08J3/12; C08J3/24; C08K5/00; C08L101/14; A61F13/53; C08K3/30; A61L15/24; A61L15/60

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2004-527620 A (NIPPON SHOKUBAI CO., LTD.) 09 September 2004 (2004-09-09)<br>entire text | 1-8 |
| A | JP 3-287870 A (MITSUBISHI PETROCHEM CO LTD) 18 December 1991 (1991-12-18)<br>entire text | 1-8 |
| A | CN 111793226 A (SHANDONG NOLL BIOSCIENCE CO., LTD.) 20 October 2020<br>(2020-10-20)<br>claims | 1-8 |
| A | JP 3-93616 A (KYOWA KAKO KK) 18 April 1991 (1991-04-18)<br>entire text | 1-8 |

☐ Further documents are listed in the continuation of Box C.     ☑ See patent family annex.

|  |  |  |  |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 June 2023** | **13 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/012460**

| Patent document cited in search report | | | Publication date (day/month/year) | Patent family member(s) | | | Publication date (day/month/year) |
|---|---|---|---|---|---|---|---|
| JP | 2004-527620 | A | 09 September 2004 | US 2004/0121081 A1 entire text | | | |
| JP | 3-287870 | A | 18 December 1991 | (Family: none) | | | |
| CN | 111793226 | A | 20 October 2020 | (Family: none) | | | |
| JP | 3-93616 | A | 18 April 1991 | (Family: none) | | | |

Form PCT/ISA/210 (patent family annex) (January 2015)

**EP 4 502 016 A1**

**Patent documents cited in the description**

- JP 2005029751 A